# EUROPEAN PATENT APPLICATION

(11) **EP 1 071 307 A1**
(43) Date of publication of application: **24.01.2001**
(21) Application number: 00202609.4
(22) Date of filing: 20.07.2000
(51) Int. Cl.: H04R 1/10, A61F 11/08

(54) **Hearing protector, hearing protection set and method for manufacturing a hearing protector**

(30) Priority: 21.07.1999 NL 1012664
(71) Applicant: Groeneveld Elcea B.V., 5107 RG Dongen (NL)
(72) Inventor: Collée, Ronald, 3039 LG Rotterdam (NL); Huijberts, Martinus Hendrikus Johannus, 5298 AS Liempde (NL)
(74) Representative: Prins, Adrianus Willem

(57) **Abstract**

A hearing protector comprising a sealing body of a flexible material adapted to be placed in the auditory duct of a person. The sealing body is provided with an internal, integrally moulded cavity, located, during use, within the auricle and accessible from the outside of the sealing body through an access duct for receiving therein in a form-closing and detachable manner a shaped part of a loud speaker housing or sealing cap shaped to substantially correspond to the moulded cavity, such that, during use of the hearing protector, the moulded cavity can be closed off from the outer world in a soundproof manner. The invention also relates to a hearing protection set and to a method for manufacturing a hearing protector.

## Description

The invention relates to a hearing protector, comprising a sealing body of flexible material, adapted to be placed in the auditory canal of a person.

Such a hearing protector having a sealing body which substantially seals off the auditory canal is generally known and is often referred to by those skilled in the art by the name of otoplast. Usually, the housing comprises a canal extending from an auricle side of the sealing body, located during use within the auricle in the auditory canal, to an eardrum side of the sealing body, located during use near the eardrum in the auditory canal. The sound canal serves for feeding ambient sound to the eardrum of a person in a damped manner and is often provided with a sound damping filter.

It has already been suggested to provide hearing protectors with loudspeakers. With the aid of such hearing protector having a loudspeaker, music or speech can be fed to the eardrum of the person. Furthermore, by means of such a combination, ambient sound can be fed to the eardrum of the person with a desired intensity.

In a first type of hearing protector, the sealing body is provided with a loudspeaker fixedly arranged in the interior of the flexible sealing body and disposed during use within the auricle. Although in this type of hearing protector the chance of undesired ambient sound entering is small and the hearing protection function is therefore well-guaranteed, this type of hearing protector also has a number of drawbacks. A drawback of this hearing protector is that the loudspeaker cannot be detached from the sealing body. In practice, the hearing protector is always provided with a wire for feeding the loudspeaker, which is not always experienced as desirable by the person wearing the hearing protector. Further, in case of damage to the wire and/or the loudspeaker, the hearing protector must be replaced wholly or partly. Consequently, the life span of the hearing protector may be shortened considerably, which entails high costs. This is particularly the case with hearing protectors from flexible material which are individually fitted.

In order to avoid this drawback, a second type of hearing protector has already been suggested, having an enlarged sealing body from flexible material, a part of which, extending during use outside of the auricle, has, near to its end, a milled-out cavity in which a generic earphone of the walk-man type can be detachably received. A drawback of this second type of hearing protector is that the acoustic sealing between the earphone and the sealing body is insufficiently reliable inter alia because of the external positioning of the loudspeaker. In particular with sudden movements and/or when the loudspeaker has already been detached a number of times, there is an increased risk of loss of the sound damping function. Further, ambient sound may enter into the auditory canal through openings in the earphone.

In addition, there is the chance that, e.g. when the original loudspeaker is out of order, it will be replaced by a different type of loudspeaker of seemingly the same type. Not only is it possible that with such a replacement loudspeaker the acoustic sealing is insufficient because of a somewhat different shape, thereby nullifying the hearing protecting function of the hearing protector, but also the output of the loudspeaker of the replacing type may be too high which, conversely, can cause hearing damage.

EP 0 590 698 describes a hearing protector having a sealing body made of rigid material adapted to be placed in the auditory canal of a person and having an access duct in which a filter housing can be received. The filter housing is provided with an integrally moulded snap ring for receiving in the access duct at a location upstream of the damping filter a corresponding part of a miniature loudspeaker, thus forming a non-airtight push button connection.

A drawback of this hearing protector is that the damping level of the hearing protector is influenced by whether or not the loudspeaker is present. Further, because of its upstream location, sound from the loudspeaker is heard through the filter which, particularly while listening to music, can lead to undesired deformation of the frequency image.

The object of the invention is to provide a hearing protector of the type mentioned in the preamble which does not have the above-mentioned drawbacks. To that end, the hearing protector according to the invention is characterized in that the sealing body comprises an internal, integrally moulded cavity, located, during use, within the auricle and accessible from the outside of the sealing body through an access duct, and connected to a sound canal extending to a side which during use is located on the eardrum side of the sealing body for receiving therein, in a form-closing and detachable manner, a shaped part of a loudspeaker housing or sealing cap, shaped to substantially correspond to the moulded cavity, such that, during use of the hearing protector, the moulded cavity can be closed off therewith airtightly from the surroundings outside of the auricle.

Thus, it is achieved that the hearing protector can be provided, at will, with a loudspeaker arranged in a loudspeaker housing or a sealing cap, so that, while retaining the hearing protection function, the loudspeaker can be exchanged with the sealing cap. Accordingly, the loudspeaker can be replaced when it is out of order or when the feeding wire is experienced as undesired. By applying an integrally moulded cavity, a reliable acoustic sealing can be realized in a simple manner, even when the loudspeaker and sealing cap are frequently exchanged. In particular, the surface of the moulded cavity, in that it is formed by means of a moulding operation, can be made sufficiently smooth in a simple manner, while furthermore, in a simple manner, a complex cavity shape favorable to the sealing can be realized. By application of a specifically shaped moulded cavity it can be achieved that the moulded cavity can only accommodate a correspondingly shaped type of loudspeaker whose output is adapted to the hearing protector and/or the sound source, so that hearing impairment can be prevented. What is achieved by placing the moulded cavity in the interior of the sealing body is that the moulded cavity, during use, is surrounded by sufficient material to energize the form-closure. Furthermore, the internal moulded cavity makes it possible for the loudspeaker to be placed, during use, within the auricle. What is thus achieved is that the risk of leakage of the acoustic sealing between the hearing protector and the auditory canal, as a consequence of the weight of the loudspeaker and/or forces on the feeding wire, can be reduced.

In a further advantageous embodiment, the hearing protector according to the invention is characterized in that the moulded cavity is slightly undersized in relation to the shaped part of the correspondingly shaped part of the loudspeaker and the sealing cap. Through this, a slight pressed fit can be realized, ensuring an even better acoustic seal between the sealing body and the loudspeaker and the sealing cap, respectively. Thus, particularly the durability of the sealing can be increased.

The invention also relates to a method for manufacturing a hearing protector and to a hearing protection set.

It is noted that soundproof sealing of the moulded cavity from the surroundings means should be understood to mean the sealing of the moulded cavity from sound entering directly from the surroundings into the moulded cavity and that any sound entering through the sound damping filter into the moulded cavity should not be taken into account here.

It will be clear that in this context form-closure should be understood to mean at least enclosing by means of a sealing body a projection of the shaped part extending radially inwards or outwards in relation to an axially extending carrier part, in such a way that the shaped part is secured within the moulded cavity against axial translation.

Further advantageous embodiments of the invention are described in the subclaims.

The invention will be further elucidated on the basis of an exemplary embodiment which is schematically represented in a drawing. In the drawing:
Fig. 1 shows a schematic representation of a hearing protection set; and
Fig. 2 shows a schematic cross-section of a loudspeaker housing with a projection for conducting sound waves.

The Figures are only schematic representations of preferred embodiments of the invention. In the Figures, identical or corresponding parts are designated by the same reference numerals.

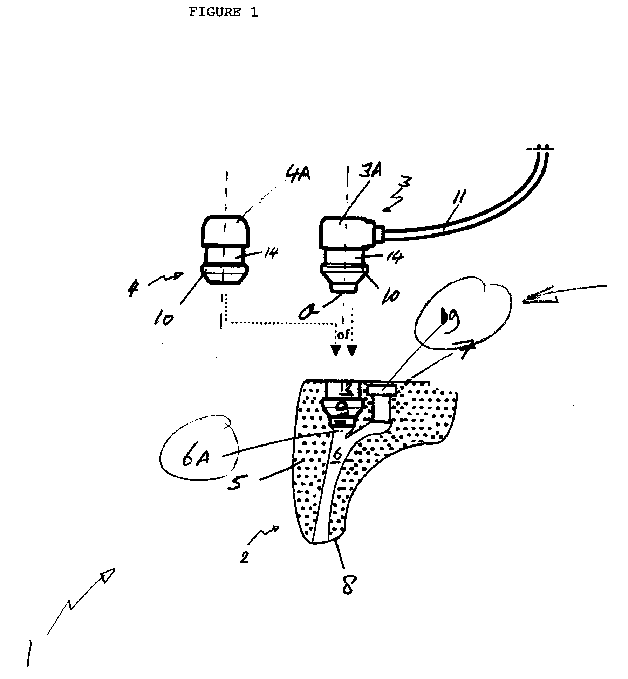

In the drawing, Fig. 1 shows a hearing protection set 1, comprising a hearing protector 2, a loudspeaker housing 3 and a sealing cap 4.

In the loudspeaker housing 3, a loudspeaker is received, such that the loudspeaker, with the exception of a sound exit opening O, is enclosed in a soundproof manner. Preferably, the loudspeaker is of the compact "Magnetic Receiver" type. Such loudspeakers are known to the skilled person and will therefore not be further elucidated here.

In the Figure, the hearing protector 2 is represented in cross-section and comprises a sealing body 5 adapted to be placed in the auditory canal of a person and made of moulded flexible material, preferably silicone material. The sealing body 5 is provided with a sound canal 6, extending from an auricle side 7 of the sealing body 5, located during use in the auditory canal adjacent the auricle, to an eardrum side 8 of the sealing body 5, located, during use, adjacent the eardrum in the sound canal 6.

In the sound canal 6 of the sealing body 5, a sound damping filter 19 is received to reduce the intensity of sound traveling through the sound canal 6 from the auricle side 7 to the eardrum side 8.

The sealing body 5 is further provided with an internal moulded cavity 9, accessible from the auricle side 7 of the exterior of the sealing body 5 via an access duct 12, for receiving therein in a form-closing and detachable manner a correspondingly shaped part 10 of the loudspeaker housing 3 or the sealing cap 4. The internal moulded cavity 9 is connected to the sound canal 6 extending, during use, to the eardrum side 8 of the sealing body 5. The moulded cavity 9 is in communication with the sound canal 6, such that the sound can travel from the loudspeaker housing 9 through the sound canal 6 to the eardrum side 8 without passing the filter 19. The moulded cavity 9 is connected to a branch 6A of the sound canal 6, this branch 6A intersecting the sound canal 6 in a flow direction from the auricle side 7 to the eardrum side 8, downstream from the sound damping filter 19.

Energized by the surrounding material, the internal moulded cavity 9 can enclose the shaped part 10 extending outwards in relation to the carrier part 14, so that, after the shaped part 10 of the loudspeaker housing 3 or the sealing cap 4 is introduced into the moulded cavity 9, at least an axial lock can be accomplished. During introduction or removal of the shaped part 10 of the loudspeaker housing 3 or the sealing cap 4 via the access duct 12, the flexible material of the sealing body 5 deforms such that the shaped part 10 can be accommodated to by the sealing body 5 and can be enclosed or released respectively.

By giving the shaped part 10 a slightly wedge-shaped form in its insertion direction, it can be achieved that insertion can be facilitated, whilst its removal can be rendered more difficult.

By slightly undersizing the moulded cavity 9 in relation to the shaped parts 10 of the loudspeaker housing 3 and the sealing cap 4, these may be received in the moulded cavity 9 in a clamped manner, thus further ensuring the acoustic sealing between the wall of the moulded cavity 9 and the wall of the shaped part 10 and enhancing the durability of the sealing. The moulded cavity 9 is connected to the sound canal 6, so that, when the loudspeaker housing 3 with its exit opening O is received in the moulded cavity 9, its sound can be fed directly to the sound canal 6, while the moulded cavity during wearing of the hearing protector is sealed soundproof with respect to the surroundings.

Advantageously, the sealing body 5 can be provided with a second moulded cavity, not shown in the Figure, in which the shaped part 10 of the sealing cap 4 can be received when the loudspeaker housing 3 with its shaped part 10 is received in the moulded cavity 9. What is thus achieved is a reduction of the risk of loss of the sealing cap 4. Naturally, it is also possible to receive the shaped part of the loudspeaker housing 3 in the second moulded cavity.

In practice, the hearing protection set can be offered in a storage package.

The hearing protector 2 can be manufactured in the customary manner by spraying a quick-curing mixture of silicone material and cross-linking agent into the auditory canal of a person and, after curing and removing the material from the auditory canal, pouring plaster around it as a core in order to obtain a lower mould part. Subsequently, after the removal of the core, in a likewise customary manner, a cover part can be placed on the lower mould part and the mould cavity thus formed can be filled with a mixture of reactants, for instance slow curing, wear-resistant silicone material and a cross-linking agent. By placing a core part whose shape substantially corresponds to the shape of the access duct 12 and the shaped part 10 on the cover part, it is achieved that, in a simple manner, by integrally moulding the core part a moulded cavity 9 of complex shape can be obtained. Optionally, the part of the loudspeaker housing 3 which during use extends within the sealing body 5 or the sealing cap 4 themselves may be used as core part. Subsequently, after the curing of the moulding material, the sealing body 5 can be removed from the mould. As will be clear from the above, because of the flexibility of the material of the sealing body, it is not necessary that the core part be self-clearing. Further, with the aid of a drilling or milling treatment, the sound canal 6 can be provided and the sound canal 6 can be connected to the moulded cavity 9.

Besides the loudspeaker, further a microphone can be received in the loudspeaker housing. Here, it is noted that it is possible to use the loudspeaker as microphone by converting sound waves received by the loudspeaker into electric signals. In such a case, loudspeaker and microphone can be embodied in the same part.

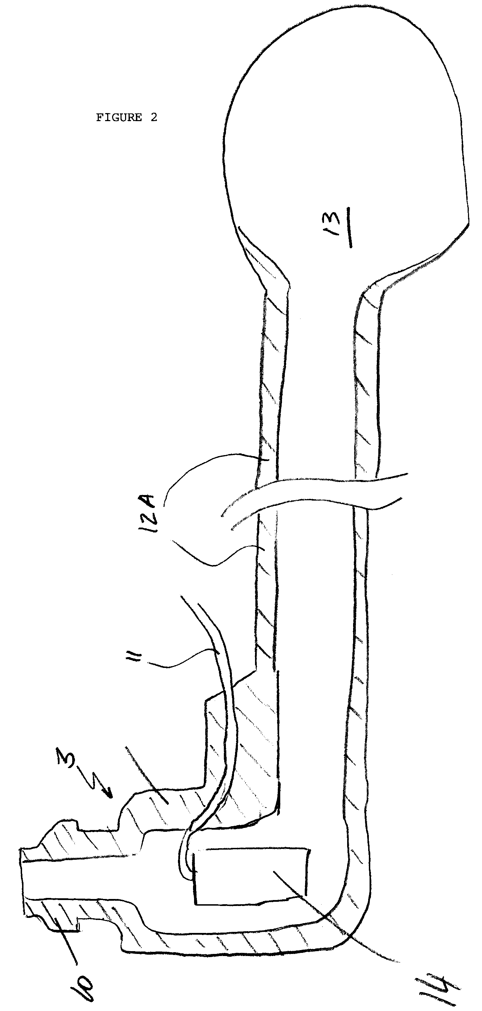

Advantageously, as is represented in Fig. 2, the loudspeaker housing 3 can be provided with a projection 12A for conducting speech signals therethrough. The projection 12A is preferably designed as a hollow tube, such that an open end 13 thereof, when the shaped part 10 of the loudspeaker housing 3, during wearing, is received in the moulded cavity 9 of the sealing body 5, can be located adjacent the mouth of the person. In the Figure it is shown that the loudspeaker housing 3 in this case is provided with an integrated loudspeaker/microphone 14. The loudspeaker/microphone is then connected via wire 11 to a transmitting/receiving device.

In this way, the hearing protector cannot only be provided at will with a loudspeaker housing with loudspeaker or with a sealing cap, but also with a loudspeaker housing with a microphone/earphone combination, with which two-way communication can be realized.

It will be clear that the projection 12A can also carry a microphone located adjacent the mouth during use and provided with a wire for conducting speech signals in electronic form.

It will further be clear that a loudspeaker housing in which in addition to a loudspeaker also a microphone is received or in which a loudspeaker/microphone combination is received can already in itself be applied in an advantageous manner.

It is noted that the invention is not limited to the exemplary embodiment described here, but that very many variants are possible.

Thus, the shaped part can be designed differently, for instance of spherical, block-shaped or star-shaped design, and several projections extending inwardly or outwardly with respect to the carrier part may be provided, for instance in order to also prevent rotation. Further, it is possible to make the part of the loudspeaker housing reaching within the sealing body and the filter substantially identical in shape, so that the loudspeaker and the filter are exchangeable and can be fitted in the moulded cavity at will. The filter may then be optionally integrated with the sealing cap. Moreover, the sealing body may be manufactured from a different kind of flexible material, for instance polyurethane.

Such variants will be immediately clear to the skilled person and are understood to fall within the scope of the invention as set forth in the following claims.

## Claims

1. A hearing protector comprising a sealing body of flexible material adapted to be placed in the auditory canal of a person, which sealing body is provided with an internal, integrally moulded cavity located, during use, within the auricle, accessible from the outside of the sealing body through an access duct and connected to a first sound canal extending to a side located during use on an eardrum side of the sealing body, for receiving therein in a form-closing and detachable manner a shaped part of a loudspeaker housing or sealing cap, shaped to substantially correspond to the moulded cavity, such that, during use of the hearing protector, the moulded cavity can be closed off therewith from the external environment in a soundproof manner.

2. A hearing protector according to claim 1, wherein the sealing body is provided with a sound canal extending from an auricle side to an eardrum side, in which sound canal a sound damping filter is received.

3. A hearing protector according to claim 2, wherein the moulded cavity is connected to the sound canal through a branch downstream of the sound damping filter.

4. A hearing protector according to any one of claims 1-3, wherein the sealing body is provided with a second moulded cavity for detachably receiving the sealing cap therein.

5. A hearing protector according to any one of claims 1-4, wherein the second moulded cavity is substantially identical in shape to the first moulded cavity, in order to be able to receive the shaped part of the loudspeaker housing or the sealing cap in a form-closing and detachable manner.

6. A hearing protector according to any one of the preceding claims, wherein the moulded cavity is designed to have a slight taper in an insertion direction, corresponding with a wedge-shaped projection of a shaped element to be received in the moulded cavity.

7. A hearing protection set, comprising a hearing protector according to any one of claims 1-6, a loudspeaker received in a loudspeaker housing and a sealing cap, the loudspeaker housing and sealing cap each being provided with a shaped part shaped to substantially correspond to the moulded cavity of the hearing protector, such that, at will, the loudspeaker housing or the sealing cap can be received in the moulded cavity in a form-closing manner and can be exchanged.

8. A hearing protection set according to claim 7, wherein the loudspeaker housing is provided with a microphone or microphone/loudspeaker combination.

9. A hearing protection set according to claim 8, wherein the loudspeaker housing is provided with a projection for conducting speech signals.

10. A hearing protection set according to any one of claims 7-9, wherein further a sound damping filter is provided having a shaped part shaped to correspond substantially to the moulded cavity, such that the sound damping filter can be received in the moulded cavity in a form-closing and detachable manner and can be exchanged at will with the loudspeaker or the sealing cap.

11. A hearing protection set according to claim 10, characterized in that the sealing cap is integrated with a sound damping filter.

12. A method for manufacturing a hearing protector, wherein in a moulding cavity for forming a flexible sealing body core parts are arranged for forming an internal moulded cavity accessible from the outside of the sealing body to be formed, for receiving therein in a form-closing and detachable manner a shaped part of a loudspeaker housing shaped to substantially correspond to the moulded cavity, such that the moulded cavity during use of the hearing protector can be sealed from the outer world therewith in a soundproof manner.
